Europäisches Patentamt

European Patent Office

Office européen des brevets

(11) Veröffentlichungsnummer: **0 406 778 A1**

## (12) EUROPÄISCHE PATENTANMELDUNG

(21) Anmeldenummer: **90112619.3**

(22) Anmeldetag: **02.07.90**

(51) Int. Cl.⁵: **A61M 5/30**

(30) Priorität: **05.07.89 HU 337389**

(43) Veröffentlichungstag der Anmeldung:
**09.01.91 Patentblatt 91/02**

(84) Benannte Vertragsstaaten:
**AT BE CH DE DK ES FR GB GR IT LI LU NL SE**

(71) Anmelder: **Gyula, Erdélyi, Dr. Dipl.-Ing.**
**Aga u. 6.**
**H-1113 Budapest(HU)**

Anmelder: **Mátrai, András, Dipl.-Ing.**
**Marogép u. 4.**
**H-1213 Budapest(HU)**

Anmelder: **Nagy, Lajos, Dipl.-Ing.**
**Fodor u. 5/7.**
**H-1126 Budapest(HU)**

(72) Erfinder: **Gyula, Erdélyi, Dr. Dipl.-Ing.**
**Aga u. 6.**
**H-1113 Budapest(HU)**
Erfinder: **Mátrai, András, Dipl.-Ing.**
**Marogép u. 4.**
**H-1213 Budapest(HU)**
Erfinder: **Nagy, Lajos, Dipl.-Ing.**
**Fodor u. 5/7.**
**H-1126 Budapest(HU)**

(74) Vertreter: **Patentanwälte Viering &**
**Jentschuraura**
**Steinsdorfstrasse 6**
**D-8000 München 22(DE)**

(54) **Nadelloses Impfgerät.**

(57) Nadelloses Impfgerät, insbesondere für Blinde und Schwachsichtige, welches einen zum Ausschießen des Impfstoffes geeignet durchbohrten Impfkopf (1) und eine Schraubenfeder (7) aufweist, die an einem Kolbenschaft (3) abgestützt ist, der in einem am Impfkopf angeschlossenen Zylinder (2) angeordnet ist. Der auf die Hautfläche senkrecht aufsetzbare Impfkopf (1) steht mit einem eine zweite Feder (8) betätigenden Federhaltergehäuse (11) durch Zwischenschalten der in der Bohrung des Federhaltergehäuses (11) angeordneten, in den Aufziehschaft (6) einrastbaren Kugel (10) in Steuerungsverbindung. Der Impfkopf (1) ist mit dem Kolben (3) gemeinsam austauschbar ausgebildet. Der Impfkopf (1) kann an einem die Impfstoffampulle aufnehmenden Adapter (19) angeschlossen werden. Das Gerät kann durch Andrücken des Impfkopfes (1) gegen die Hautfläche in Betrieb gesetzt werden.

Fig.1

EP 0 406 778 A1

## NADELLOSES IMPFGERÄT

Die Erfindung bezieht sich auf ein nadelloses Impfgerät, insbesondere für Blinde und Schwachsichtige, welches mit austauschbaren Hauptbauteilen aus Kunststoff versehen ist. Das Impfgerät ist geeignet, Insulin oder anderen flüssigen Stoff -als individuelle Impfung - in den Organismus von Menschen oder Tieren einzuführen.

Es ist bekannt, daß bei einigen Krankheiten es notwendig ist, flüssige Medikamente längere Zeit hindurch mit großer Häufigkeit in den lebenden Organismus einzuführen.

Zur Eingabe von verschiedenen Heilmitteln werden in der Mehrzahl sogenannte "einmal verwendbare" Kunststoffspritzen und Nadeln verwendet. Diese haben denjenigen großen Nachteil, daß ihr sogenannter "Totraum" groß ist; deshalb bleibt nach der Impfung eine größere Menge Heilmittel im Totraum, das heißt in der Spritze und in der Nadel zurück.

Die mittels Nadel durchgeführte Medikamenteneingabe kann mit Infektion, Entzündung, Schmerzen verbunden sein. Aus diesem Grund erfolgten Anstrengungen zur Schaffung derartiger nadelloser Impfgeräte, bei denen die Vorbedingung für das Funktionieren eine durch Preßluft, Gaspatrone oder Öldruck vorgespannte Feder gewährleistet.

Auf diesem Prinzip funktioniert das in der US-A-4.596.556 beschriebene Impfgerät, welches mit einer Kohlendioxyd-Gaspatrone versehen ist. Der Nachteil dieses Gerätes besteht darin, daß die Gasausströmungsgeschwindigkeit, und so auch die Einschußkraft nicht geregelt werden können. Ein weiteres Problem bedeutet, daß das gute Funktionieren des Gerätes durch gleichermaßen gefüllte Gaspatronen vorbedingt ist. Ein weiterer Nachteil des Gerätes liegt darin, daß es ein Mischen der Impfstoffe nicht löst.

Das in der DE-A-3 328 173 beschriebene Gerät löst zwar einen Teil der Grundfunktion des nadellosen Impfgerätes, aber sein wesentlicher Nachteil besteht in seiner Kompliziertheit, infolgedessen das Gerät nur mit spezieller Technologie hergestellt werden kann, also seine Produktionskosten sind hoch. Ein weiterer Nachteil des Gerätes liegt darin, daß die mit dem Impfstoff in Berührung kommenden Flächen häufig sterilisiert werden müssen. Da bei dieser Ausführungsform der vordere Teil des Kolbens nicht desinfiziert werden kann, besteht bei diesem Gerät eine große Infizierungsgefahr. Das Gerät hat weiterhin denjenigen Nachteil, daß seine Abzugskonstruktion mit Knopfdruck ausgebildet ist; so besteht die Gefahr, daß es auch in einer von der Senkrechten zur Hautfläche abweichenden Lage abgedrückt werden kann, was die Beschädigung der Hautfläche und eine schlechte Impfung verursachen kann. Aus diesem Grund können Blinde und Schwachsichtige dieses Gerät nicht verwenden.

Bei den in der DE-A-19 44 006 und DE-A-31 15 376 beschriebenen Ausführungsformen ist das Funktionieren durch Hochdruckgas sichergestellt, aber die Regelung der Einschußtiefe und das Mischen der Impfstoffe ist nicht einmal bei diesen Geräten gelöst.

Eine von den obigen abweichende Entwicklungstendenz wird durch das Impfgerät nach der FR-A-2 549 729 repräsentiert, welches ein aus einem Heilmittelraum und einem Druckraum zusammengesetztes zylindrisches Gehäuse hat. Das Gerät besteht aus das Heilmittel in den Heilmittelraum führenden Elementen, aus der das Heilmittel ausspritzenden Öffnung, aus dem im Heilmittelraum verschiebbaren Kolben und weiterhin aus Betätigungselementen.

Der grundsätzliche Mangel dieser Ausführungsform besteht darin, daß die Einschußtiefe nicht verändert werden kann; so besteht keine Möglichkeit, die Druckkraft bei unterschiedlichen Hautarten zu verändern. Ein weiterer Nachteil dieses Gerätes ist, daß mehrere Impfstoffe nicht in beliebiger Proportion zwecks Einschießbarkeit mit nur einer Impfung vermischt werden können.

Für das Impfgerät nach der EP-B-01 14 792 ist es charakteristisch, daß es nur für subcutane Impfung geeignet ist. Das Gerät besteht aus einer Düse, der das flüssige Heilmittel einsaugenden und ausspritzenden Einheit, den die Heilmitteldosis einstellenden Elementen und dem die Ampullen tragenden Teil. Die Mangelhaftigkeit dieser Ausführungsform besteht darin, daß die Menge des in den Impfraum einsaugbaren Impfstoffes zwar eingestellt werden kann, aber bei dem Einsaugen von mehreren Impfstoffen nacheinander die Möglichkeit der kontinuierlichen Einstellung, das heißt das Mischen der Impfstoffe im Impfraum nicht sichergestellt ist.

In der EP-B-01 19 286 ist ein mittels eines Druckmediums betätigtes Impfgerät beschrieben, welches einen sich im zylindrischen Gehäuse bewegenden, mit einem Saugkolben zusammenwirkenden Arbeitskolben, einen am Gerätekörper gelenkartig angeschlossenen Handhebel und eine die Bewegung des Arbeitskolbens in Abhängigkeit vom Druckwert auslösende Arretierungskonstruktion aufweist.

Der grundsätzliche Nachteil dieser Ausführungsform besteht darin, daß sie das Einsaugen von mehreren Impfstoffen in den Impfraum (kalibrierbar und vermischt) nicht sicherstellt, weil das Einsaugen der eingestellten Menge entsprechend nur in einem Takt erfolgt. Ein weiterer Nachteil dieser Ausführungsform liegt darin, daß zum Funktionieren des Impfgerätes äußere Energie erforderlich ist, ohne die das Impfgerät betriebsunfähig wird. Diese Tatsache verursacht, daß das Impfgerät ortsgebunden ist.

Bei den aufgeführten Impfgeräten bestehen die folgenden gemeinsamen Nachteile: die Einschußtiefe kann nicht geregelt werden; es besteht keine Möglichkeit zum Mischen von Impfstoffen und dies bedeutet, daß die Eingabe von zwei Insulinen verschiedenen Wirkungsgehaltes (Actrapid und Ultrabard) in den Organismus nur mit zwei Impfungen und an zwei verschiedenen Stellen durchgeführt werden kann.

Von den Produkten derselben Firma können die Insuline mit verschleppter Wirkung zwecks Verstärkung ihres Wirkungsbeginns im allgemeinen mit schnell wirkenden Insulinen vermischt werden, ohne daß sie ihre gegenseitige Wirkung beeinflussen, also verkürzen oder verschleppen würden.

Zielsetzung unserer Erfindung ist es, ein derartiges nadelloses, die Einschußtiefe regelndes, mit austauschbaren Hauptbauteilen aus Kunststoff versehenes, zum Impfen - z.B. von Insulin - geeignetes Impfgerät ohne Abzugskonstruktion zu schaffen, welches die aufgezählten Mangelhaftigkeiten nicht aufweist, auf leichte Weise und mit großer Genauigkeit und Sicherheit sogar durch blinde oder schwachsichtige Zuckerkranke betätigt werden kann, weiterhin geringe Abmessungen und ein geringes Gewicht hat.

Nachdem die beiden Hauptbauteile, der Kolben und der Zylinder, vorgefertigte und sterilisierte Produkte sind, erübrigt sich die Sterilisation je Woche oder je zwei Wochen, weil sie leicht austauschbar und vorangehend schon sterilisiert sind.

Die gestellte Aufgabe wird erfindungsgemäß mit einer Ausführungsform erfüllt, die dadurch gekennzeichnet werden kann, daß die drei Hauptteile des Impfgerätes das Funktionieren des Gerätes sicherstellen, die Impfeinheit und die Betätigungseinheit in einer Einheit enthalten sind, und mit der Vorspannung der den Druck erzeugenden Schraubenfeder gleichzeitig auch das Auffüllen des Kunststoff-Impfstoffzylinders auf gut kontrollierbare Weise erfolgt.

Die gewünschte Einschußtiefe kann durch Verdrehen des Gerätekörpers gegenüber dem zur Vorspannung der Schraubenfeder dienenden Gerätegehäuse eingestellt und leicht kontrolliert werden.

Die Erfindung wird nachstehend anhand einer bevorzugten Ausführungsform mit Hilfe der Zeichnung näher erläutert. Es zeigen:

Fig. 1 das erfindungsgemäße Impfgerät im Längsschnitt,

Figuren 2, 3 und 4 die Profilenden der am erfindungsgemäßen Impfgerät anschließbaren Adapter

Fig. 5 einen am erfindungsgemäßen Impfgerät anschließbaren Adapter und

Fig. 6 - das erfindungsgemäße Impfgerät in Vorderansicht.

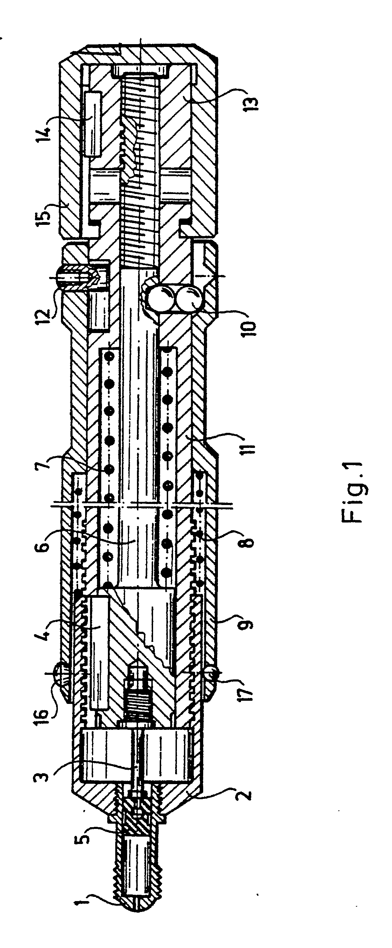

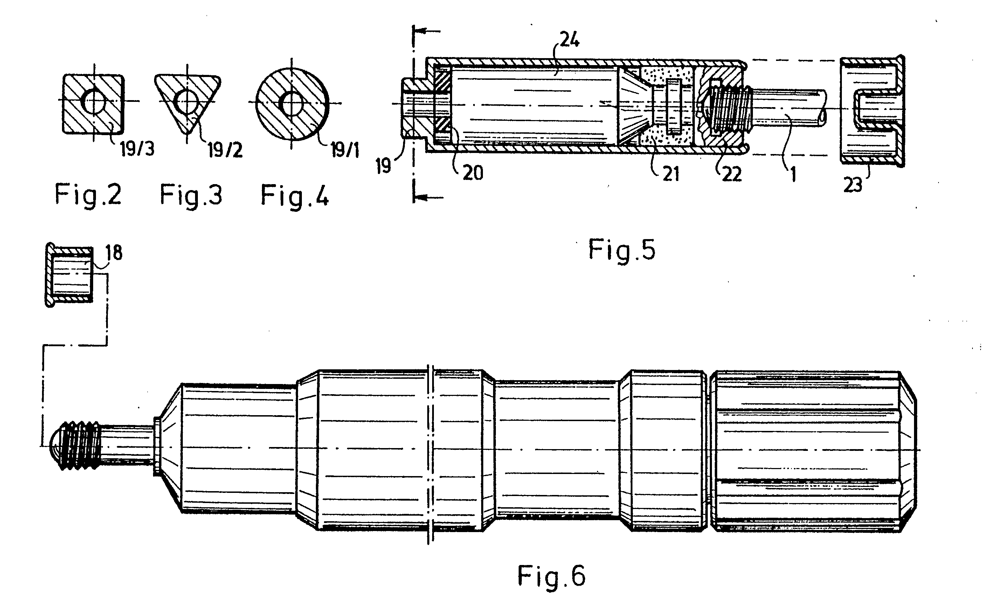

Der Funktionsteil des Gerätes ist im Gerätegehäuse 9 und im Federhaltergehäuse 11 enthalten. Aus Fig. 5 ist ersichtlich, daß nach Einschrauben des Außengewindes des Impfkopfes 1 in den Adapter 19 bis zum Adaptergewindeende 22 und nach Andrücken der am Abstandshalter 20 angeschlagenen Ampulle 24 der Adapter 19 mittels der Zentrierungsdichtung 21 an den Impfkopf 1 angeschlossen ist, wonach durch weiteres Drehen des Adapters 19 gegenüber dem Gerätegehäuse 9 in gleicher Richtung über den Keil 4 der Aufziehschaft 6 in der Mutter 13 zurückgeschraubt wird und dadurch der Impfstoff in den Kalibrierungszylinder 2 eingesaugt wird und die Schraubenfeder 7 gespannt wird. Dieser Vorgang wird spätestens beendet, wenn der Keil 4, wie es in Fig. 1 zu sehen ist, an dem Federhaltergehäuse 11 anschlägt.

In dieser Lage ist die weitere Bewegung durch die Doppelkugel 10 arretiert.

Die Aufziehoperation ist durch die Mutter 13 gesichert, die über den Keil 14 am Griff 15 drehfest angeschlossen ist; so kann sie sich an dem am Aufziehschaft 6 ausgebildeten Gewinde bewegen.

Bei dem Einsaugen des Impfstoffes mittels Verdrehens des Kalibrierungszylinders 2 gegen den Uhrzeigersinn zeigt die am Kalibrierungszylinder 2 ausgebildete Maßteilungseinheit an der Lupe 16 die Impfstoffmenge.

Bei einem Mischen von Impfstoffen (Actrapid und Ultrabard) soll der Adapter 19 nach der ersten Einstellung mit der in diesem befindlichen Ampulle 24 gemeinsam ausgetauscht werden.

Blinde und Schwachsichtige können die Güte des Einsaugens des Impfstoffes bzw. die Impfstoffart mittels Fingerfühlung der in den Figuren 2, 3 und 4 dargestellten Profilenden der Adapter 19/1, 19/2 und 19/3 wahrnehmen.

Das Maß des Einsaugens wird durch die Klangwirkung bei der Bewegung der Kugel 17 in der Rastteilung akustisch angezeigt.

Für die Vorbereitung zum Einschießen wird der Griff 15 gegenüber dem Gehäuse 9 gegen den Uhrzeigersinn gedreht, so daß die Mutter 13 am Gewinde des Aufziehschaftes 6 bis zum Anschlag zurückbewegt wird.

Bei der Eingabe des Impfstoffes in den Organismus wird das Gerätegehäuse 9 senkrecht zur Hautfläche gehalten und der Impfkopf 1 mit geringem Druck gegen die Hautfläche gedrückt.

Dadurch schiebt der am Impfkopf 1 über ein Gewinde angeschlossene Kalibrierungszylinder 2 das Federhaltergehäuse 11 gegen die Federkraft der Feder 8 nach hinten.

Die Bewegung ist durch die am Federhaltergehäuse 11 ausgebildete Keilnut gesichert, in welche die Keilschraube 12 eingreift.

Beim Zurückbewegen bewegt sich die Doppelkugel 10 bei Erreichen der Kegelvertiefung im Gerätegehäuse 9 radial nach außen und dadurch wird der Aufziehschaft 6 freigegeben und das Vorwärtsspringen des Aufziehschaftes 6 - der von der Schraubenfeder 7 umgeben ist - und damit des Kolbens 3 ermöglicht, so daß der eingesaugte Impfstoff aus dem Impfkopf 1 mit hohem Druck unter die Hautfläche gespritzt wird.

Bei dem erfindungsgemäßen Gerät ist die Sterilisierung je zwei Wochen erübrigt, weil der eine Vorteil unserer Ausführungsform eben darin liegt, daß der Impfkopf 1 und die Dichtung 5 nach einem Zweiwochengebrauch weggeworfen bzw. ausgetauscht werden können. Der Impfkopf 1 kann mittels Ausschrauben aus dem Kalibrierungszylinder 2 und Einsetzen des neuen, in sterilisiertem Kunststoffbeutel eingepackten Impfkopfes ohne Berührung mit der Hand ausgetauscht werden.

Die Dichtung 5 kann durch Abziehen vom Kolben 3 mit der Hand und Aufsetzen der neuen, in sterilisiertem Kunststoffbeutel eingepackten Dichtung ausgetauscht werden.

Die sterile Lagerung des Gerätes und des Adapters 19 in der Zeitperiode zwischen zwei Auffüllungen ist durch die in Fig. 6 dargestellte Impfkopfschließkappe 18 und Adapterschließkappe 23 nach Fig. 5 sichergestellt.

Die Erfindung ist nicht auf die beispielsweise Ausführungsform eingeschränkt.

Ein Vorteil des erfindungsgemäßen Impfgerätes besteht auch darin, daß die mit dem Impfstoff in Berührung gelangenden Flächen - nach dem Vorbild der wegwerfbaren Spritzen -ausgetauscht, weggeworfen werden können; so besteht keine Infektionsgefahr. Das Gerät wird durch Drücken des Impfkopfes 1 auf die Hautfläche in Betrieb gesetzt, auf diese Weise ist das zur Hautfläche senkrechte Einspritzen sichergestellt. Das Gerät ist auf einfache, leichte Weise herstellbar.

## Ansprüche

1. Nadelloses Impfgerät insbesondere für Blinde und Schwachsichtige, zum Ausschiessen des Impfstoffes mittels in der Wirkungslinie einer geregelt vorgespannten Schraubenfeder (7) angeordneten Kolbens (3), dadurch gekennzeichnet, daß es ein Gerätegehäuse (9), einen darin geführten Kalibrierungszylinder (2) und zwischen diesen eine Feder (8) hat, weiterhin ein drehfest geführtes Federhaltergehäuse (11), darin einen Aufziehschaft (6) und zwischen diesen die Schraubenfeder (7) aufweist, zu deren Spannen eine mittels eines Griffes (15) verdrehbare Mutter (13) dient, die in ein am Aufziehschaft (6) ausgebildetes Gewinde eingreift, und zur Arretierung des Aufziehschaftes (6) eine Doppelkugel (10) angeordnet ist.

2. Nadelloses Impfgerät nach Anspruch 1, dadurch gekennzeichnet, daß das Gerätegehäuse (9) mit einer Anzeigekugel (17) versehen ist.

3. Nadelloses Impfgerät nach Anspruch 1 oder 2, dadurch gekennzeichnet, daß an dem dem Gewindeende des Aufziehschaftes (6) gegenüberliegenden Ende ein Kolben (3) mittels einer lösbaren Verbindung angeschlossen ist.

4. Nadelloses Impfgerät nach einem der Ansprüche 1 bis 3, dadurch gekennzeichnet, daß am Federhaltergehäuse (11) ein Kalibrierungszylinder (2) angeschlossen ist.

5. Nadelloses Impfgerät nach einem der Ansprüche 1 bis 4, dadurch gekennzeichnet, daß der Kalibrierungszylinder (2) zum Anschließen des Impfkopfes (1) geeignet ausgebildet ist.

6. Impfkopf des nadellosen Impfgerätes insbesondere für Blinde und Schwachsichtige, dadurch gekennzeichnet, daß der mit Dichtung (5) montierte, aus durchsichtigem Material gefertige Impfkopf (1) einen Anschließteil mit lösbarer Verbindung und weiterhin eine auf die Zusammensetzung des im Impfkopf (11) befindlichen Impfstoffes hinweisende geometrisch ausgebildete, zum Halten von Scheiben dienende Fläche aufweist.

7 . Impfkopf nach Anspruch 6, dadurch gekennzeichnet, daß der mit lösbarer Verbindung ausgebildete Teil des Impfkopfes (1) gewindeartig ausgebildet ist.

8. Adapter zum Aufsaugen von Impfstoff in den Impfkopf, dadurch gekennzeichnet, daß das Gehäuse des Adapters (19) und dessen Profilenden (19/1, 19/2, 19/3) in auf die Zusammensetzung des im Adapter (19) befindlichen Impfstoffes hinweisender veränderbarer Form ausgebildet sind.

Fig.1

EP 0 406 778 A1

Fig.5

Fig.6

Fig.2 Fig.3 Fig.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 276 158 (ADVANCED MEDICAL TECHNOLOGIES INC.) * Figuren 1-6; Spalte 3, Zeile 60 - Spalte 4, Zeile 6; Spalte 4, Zeilen 11-13,30-37,44-47,55,56; Spalte 5, Zeilen 9-43 * --- | 1-5,7 | A 61 M 5/30 |
| A | US-A-4 662 878 (LINDMAYER) * Figuren 1-5; Spalte 1, Zeile 42 - Spalte 2, Zeile 12 * --- | 1 | |
| A,D | DE-A-3 328 173 (PRECI-TECH LTD) * Figuren 3,4,6,7; Seite 12, Zeilen 12-24; Seite 13, Zeilen 23-31; Seite 14, Zeilen 6-26; Seite 15, Zeilen 17-28; Seite 17, Zeilen 4-6 * --- | 1 | |
| A,D | EP-A-0 114 792 (SICIM S.p.A.) * Figur 1a; Patentanspruch 1 * --- | 1 | |
| A | GB-A- 995 605 (THE AMALGAMATED DENTAL CO., LTD) * Figur 1; Seite 3, Zeilen 22-28,116,117; Seite 4, Zeile 30 * --- | 1 | RECHERCHIERTE SACHGEBIETE (Int. Cl.5) A 61 M |
| A | US-A-4 208 983 (BUCKLEY) * Figuren 1,4; Spalte 2, Zeilen 56-68 * ----- | 8 | |

Der vorliegende Recherchenbericht wurde für alle Patentansprüche erstellt

| Recherchenort | Abschlußdatum der Recherche | Prüfer |
|---|---|---|
| DEN HAAG | 23-10-1990 | SEDY, R. |